# EUROPEAN PATENT SPECIFICATION

(11) **EP 1 240 880 B1**
(45) Date of publication and mention of the grant of the patent: **05.10.2011**
(21) Application number: 02076342.1
(22) Date of filing: 12.03.2002
(51) Int. Cl.: A61F 13/08

(54) **Therapeutic elastic envelope**
Therapeutische elastische Hülle
Enveloppe élastique à usage thérapeutique

(30) Priority: 15.03.2001 NL 1017609
(43) Date of publication of application: 18.09.2002
(73) Proprietor: Stolk, Robert, 1747 GM Tuitjenhorn (NL)
(72) Inventor: Stolk, Robert, 1747 GM Tuitjenhorn (NL)
(74) Representative: Metman, Karel Johannes

(56) References cited:
- EP-A- 0 272 989
- FR-A- 2 633 512
- US-A- 2 218 413
- US-A- 3 386 270

## Description

The present invention relates to a therapeutic elastic envelope, made from elastic textile and dimensioned such that, in use, at least the lower leg of a user is surrounded.

Therapeutic elastic envelopes in the form of stockings are used for example in the treatment of patients suffering from venous and lymphatic leg diseases. In the starting phase of the treatment of these diseases the phlebologist aims at freeing the leg from oedema as good and quickly as possible. This is happening by compression of the leg by means of bandages. In principle there are two types of bandages: elastic and non-elastic bandages. During the treatment phase the non-elastic bandages appear to be the most effective ones: when the patient is walking the non-elastic bandage will deliver forceful pressure pulses to the underlying leg segments as a result of the contraction of the underlying muscles.

The phlebologist summarises the ideal effect of the bandage as follows: "The bandage must have a low rest pressure and a high working pressure". The walking action of the patient is consequently a requirement to cause said compression. One therefore speaks of ambulant compression therapy.

The therapeutic elastic stocking is preferably used after the phlebologist has freed the leg of oedema by means of bandages. One of the tasks of the pressure pattern of the stocking is to compress the venous bed and consequently to cause a better flow of blood therethrough. The veins are compressed, the valves function better and reoccurrence of oedema is counteracted.

The object of the invention is to provide an improved theraupatic elastic envelope.

For this purpose the envelope according to the invention is characterised by the features of the characterizing portion of claim 1. It is shown that a therapeutic elastic envelope having such less elastic portion may generate high pressure pulses on the underling leg segment. Apparently the circumferential tension is not leveled due to the friction between the skin and the textile of the envelope. The action of this envelope thereby approaches the one of the non-elastic bandage. The ability of the therapeutic elastic envelope according to the invention to prevent formation of liquid is significantly better then with conventional therapeutic elastic envelopes, in particular stockings. It is noted that EP-B-0 272 989 discloses a stocking having a less elastic portion, but the purpose of this less elastic portion is not clear and this document also does not teach anything about the position of this portion. FR 2 633 512 discloses a therapeutic elastic bandage for application on joints, such as ankle, knee and elbow. US 3,386,270 discloses a knit-rubber surgical hosery having a bending area devoid of rubber, for the purpose of facilitating bending of the part of the anatomy encased by the article and to eliminate the tendency toward chafing in said portion.

According to the invention the less elastic portion must be positioned on the rear side of the stocking because the variations in circumference during walking mainly take place there, this due to the expansion of the calf muscle. This calf muscle actually consists of two muscles: the Gastrocnemius and a Soleus muscle both merging into the Achilles tendon which lifts the foot and consequently the body during walking.

According to the invention the less elastic portion, which may be substantially inelastic, is positioned on the stocking such, that, in use, it extends from ca. 1/3 up to ca. 2/3 of the height of the lower leg of the user where, during use of the stocking on the leg, expanding muscles are present below the skin of the user, and preferably along circa 20-40% of the circumference at a maximum. In that case high pressure pulses are generated on the leg in a reliable manner and on the right places.

The invention will hereafter be elucidated with reference to the drawing showing very schematically an embodiment of the therapeutic elastic envelope according to the invention in the form of a stocking.

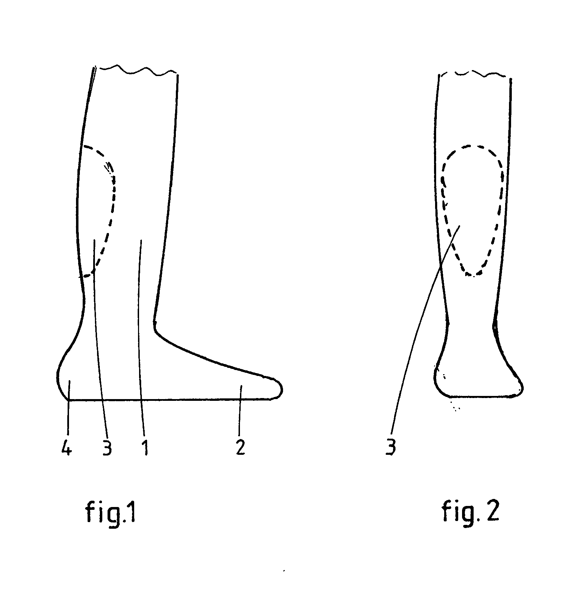

Fig. 1 is a side view of a therapeutic elastic stocking according to the invention.
Fig. 2 is a rear view of the stocking of fig. 1.

The drawing shows an embodiment of the envelope according to the invention in the form of a therapeutic elastic stocking which comprises a leg part 1 and a foot part 2. The leg part 1 will extend in use around the lower leg of the patient, but may possibly also surround the knee and a part of the upper leg. In the drawing the stocking is cut-off at the upper side of the lower leg.

The stocking is manufactured from an elastic textile, such as knit-wear, fabric or non-woven and comprises, at least in the circumferential direction of the stocking, elastic threats which provide the fabric with elasticity.

According to the invention the stocking, in particular the leg part 1 thereof, comprises a less elastic portion 3 positioned on the rear side of the stocking, that is on the same side as the heel 4 of the stocking.

The less elastic portion 3 is positioned on such height and with such size that in use it extends from circa 1/3 up to circa 2/3 of the height of the lower leg of the user. In circumferential direction the less elastic portion will extend preferably along circa 20-40% of the circumference at a maximum. As a result, the muscles to which the pressure pulses must be exerted are sufficiently covered. Preferably, the less elastic portion is slightly leaf-shaped, that is the upper side of the less elastic portion 3 is slightly wider, while it is slightly tapered on the upper and lower sides. This is adapted to the extent of the underlying leg muscles, in this case the calf muscle.

Preferably, the less elastic portion is substantially non-elastic, in particular in circumferential direction of the leg part 1, in which it is preferred to integrate the portion 3 into the textile of the leg part 1 of the stocking. The less elastic portion 3 is then formed of a textile of less elastic, or inelastic or hardly elastic material, respectively, at least in circumferential direction of the stocking. Of course a portion may also be made less elastic or inelastic in another way, such as by stabilizing the textile, for example, by applying a stretch preventing layer or by a technical process, such as heating. In case of an inelastic textile it is simple to reduce the elasticity only in circumferential direction by knitting or weaving with less elastic threads in that direction. The elasticity in the longitudinal direction may then be kept substantially equal to that of the surrounding textile of the envelope.

The portion 3 may extend through a greater height and greater and smaller width as mentioned, depending on the patient and the therapy as used.

From the foregoing it will be clear that the invention provides a therapeutic elastic envelope which is very well suited for use in the ambulant compression therapy and combines the advantages of the inelastic bandage and the elastic envelope.

## Claims

1. Therapeutic elastic envelope in the form of a stocking, made from elastic textile and dimensioned such that, in use, at least the lower leg of a user is surrounded, wherein the stocking comprises a less elastic portion, **characterized in that** the less elastic portion is positioned on the rear side of the stocking such that, in use, it extends from ca. 1/3 up to ca. 2/3 of the height of the lower leg of the user where, during use of the stocking on the leg, expanding muscles are present below the skin of the user.

2. Envelope according to claim 1, wherein the less elastic portion is substantially inelastic.

3. Envelope according to one preceding claims, wherein the less elastic portion is substantially leaf shaped.

4. Envelope according to the one preceding claims, wherein the less elastic portion is integrated in the knitting or fabric of the envelope.

5. Envelope according to the claim 4, wherein the less elastic portion is formed of knit-wear or fabric containing little elastic material.

6. Envelope according to the claim 5, wherein the little elastic material of the knit-wear or fabric extends in the circumferential direction of the envelope only.

7. Envelope according to one preceding claims, which, in use, extends from the toe up to the upper side of the lower leg.

## Patentansprüche

1. Therapeutische elastische Umhüllung in Form eines Strumpfs aus elastischem Gewebe und derart dimensioniert, dass bei Gebrauch wenigstens der Unterschenkel eines Benutzers umgeben ist, wobei der Strumpf einen weniger elastischen Abschnitt umfasst, **dadurch gekennzeichnet, dass** der weniger elastische Abschnitt derart auf der Rückseite des Strumpfs positioniert ist, dass er sich bei Benutzung im Wesentlichen von 1/3 bis zu 2/3 der Höhe des Unterschenkels des Benutzers erstreckt, wo sich während der Benutzung des Strumpfs am Bein sich ausdehnende Muskeln unter der Haut des Benutzers befinden.

2. Umhüllung nach Anspruch 1, wobei der weniger elastische Abschnitt im Wesentlichen unelastisch ist.

3. Umhüllung nach einem der vorangehenden Ansprüche, wobei der weniger elastische Abschnitt im Wesentlichen blattförmig ist.

4. Umhüllung nach einem der vorangehenden Ansprüche, wobei der weniger elastische Abschnitt in die Maschen oder die Fasern der Umhüllung eingearbeitet ist.

5. Umhüllung nach Anspruch 4, wobei der weniger elastische Abschnitt aus Maschenwaren oder Fasern mit gering elastischem Material gebildet ist.

6. Umhüllung nach Anspruch 5, wobei sich das wenig elastische Material der Maschenware oder Fasern nur in Umfangsrichtung der Umhüllung ausdehnt.

7. Umhüllung nach einem der vorangehenden Ansprüche, wobei sie sich bei Benutzung von den Zehen bis zur Oberseite des Unterschenkels ausdehnt.

## Revendications

1. Enveloppe élastique thérapeutique sous forme d'un bas, réalisée en textile élastique et dimensionnée de sorte qu'en utilisation, au moins la jambe inférieure d'un utilisateur soit entourée, où le bas comprend une partie moins élastique, **caractérisée en ce que** la partie moins élastique est positionnée sur le côté arrière du bas de sorte qu'en utilisation, elle s'étende d'environ 1/3 jusqu'à environ 2/3 de la hauteur de la jambe inférieure de l'utilisateur où, durant l'utilisation du bas sur la jambe, des muscles dilatés sont présents sous la peau de l'utilisateur.

2. Enveloppe selon la revendication 1, dans laquelle la partie la moins élastique est essentiellement inélastique.

3. Enveloppe selon l'une des revendications précédentes, dans laquelle la partie la moins élastique est essentiellement en forme de feuille.

4. Enveloppe selon l'une des revendications précédentes, dans laquelle la partie la moins élastique est intégrée dans le tricot ou le tissu de l'enveloppe.

5. Enveloppe selon la revendication 4, dans laquelle la partie la moins élastique est réalisée en tricot ou en tissu contenant un peu de matière élastique.

6. Enveloppe selon la revendication 5, dans laquelle le peu de matériau élastique du tricot ou du tissu s'étend dans la direction circonférentielle de l'enveloppe uniquement.

7. Enveloppe selon l'une des revendications précédentes, qui, en utilisation, s'étend de l'orteil jusqu'au côté supérieur de la jambe inférieure.
